# EUROPEAN PATENT APPLICATION

(11) **EP 4 372 087 A1**
(43) Date of publication of application: **22.05.2024**
(21) Application number: 22853368.3
(22) Date of filing: 29.07.2022
(51) Int. Cl.: C12N 15/113, A61K 31/7088, A61P 35/00, A61K 48/00

(54) **COMPOSITION FOR PREVENTION OR TREATMENT OF LIVER CANCER, COMPRISING MODIFIED RT-LET7 AS ACTIVE INGREDIENT**

(30) Priority: 06.08.2021 KR 20210103567; 20.10.2021 KR 20210140386; 28.07.2022 KR 20220093739
(71) Applicant: Neornat, Seocho-gu Seoul 06591 (KR)
(72) Inventor: NAM, Suk-Woo, Seoul 06600 (KR); YANG, Hee-Doo, Seoul 04607 (KR)
(74) Representative: Isarpatent
(86) International application number: PCT/KR2022/011190
(87) International publication number: WO 2023/013990

(57) **Abstract**

The present invention relates to a pharmaceutical composition for prevention or treatment of liver cancer, comprising, as an active ingredient, modified RT-LET7 or modified RT-LET7 with which a liver cell-targeted moiety is combined. Compared to a conventional RT-LET7, which is antisense microRNA (AS-miRNA) for inhibiting Let-7i-5p, the modified RT-LET7 according to the present invention has effects of increasing the inhibition of Let-7i-5p expression, increasing the inhibition of liver cancer cell growth, increasing TSP1 expression, and increasing phagocytosis activity of macrophages. In addition, the liver cell-targeted moiety is combined with the modified RT-LET7 so as to improve delivery ability to liver cancer cells and remarkably improve the effectiveness of liver cancer treatment, and thus the modified RT-LET7 can be effectively used in a pharmaceutical composition for prevention or treatment of liver cancer, or as a checkpoint inhibitor for prevention or treatment of CD47-positive liver cancer.

## Description

### [Technical Field]

The present invention relates to a pharmaceutical composition for preventing or treating liver cancer, and specifically, to a pharmaceutical composition for preventing or treating liver cancer including modified RT-LET7, which is an expression inhibitor of Let-7i-5p, or modified RT-LET7 loaded with a delivery system as an active ingredient.

### [Background Art]

Liver cancer is the fifth most common cancer worldwide, but an aggressive cancer with the third highest mortality rate (Ahn J, Flamm SL Hepatocellular carcinoma Dis Mon 2004;50:556-573). Therapeutic surgery is available for only 15% to 25% of patients, and most liver cancer patients die within a relatively short period of time due to locally advanced or metastatic diseases (Roberts LR, Gores GJ Hepatocellular carcinoma: molecular pathways and new therapeutic targets, Semin Liver Dis, 2005; 25:212-225). Hepatitis B virus, hepatitis C virus, aflatoxin B1, etc. are well known as major causes of liver cancer. However, the overall survival rate of liver cancer patients has not increased significantly over the past 20 years, and the mechanisms of development and progression of liver cancer are still unknown well (Bruix J, et al., Focus on hepatocellular carcinoma, Cancer Cell, 2004; 5:215-219). So far, molecular targeted therapy has been effective in the treatment of mature liver cancer (Shen YC, Hsu C, Cheng AL Molecular targeted therapy for advanced hepatocellular carcinoma: current status and future perspectives, J Gastroenterol; 45:794-807), but it is unclear how these genetic changes cause the clinical characteristics observed in individual patients with liver cancer.

Histone deacetylases (HDACs) may be often attached to gene promoters by corepressors or multi-protein transcriptional complexes, and regulate transcription through chromatin modification rather than directly binding to DNA in the gene promoters (Thiagalingam S, Cheng KH, Lee HJ, Mineva N, Thiagalingam A, Ponte JF Histone deacetylases: unique players in shaping the epigenetic histone code Ann N Y Acad Sci 2003;983:84-100). There are 18 encrypted human HDACs, which are classified into Class I (HDACs 1, 2, 3, and 8), Class II (HDACs 4, 5, 6, 7, 9, and 10), Class III (SIRTs 1-7), and Class IV (HDAC11) enzymes (Yang XJ, Seto E The Rpd3/Hda1 family of lysine deacetylases: from bacteria and yeast to mice and men Nat Rev Mol Cell Biol 2008;9:206-218). It is known that both histone acetyltransferases and HDACs are involved in cell proliferation, differentiation, and cell cycle regulation (Witt O, Deubzer HE, Milde T, Oehme I HDAC family: What are the cancer relevant targets Cancer Lett 2009;277:8-21). In addition, it has been reported that pathological activity and deregulation of HDACs may cause several diseases, such as cancer, immune diseases, and muscular dystrophy (Yang XJ, Seto E HATs and HDACs: from structure, function and regulation to novel strategies for therapy and prevention Oncogene 2007;26:5310-5318). HDAC6 is a member of the class Ilb family of HDACs, acts as cytoplasmic deacetylase associated with microtubules (MTs), and deacetylates α-tubulin (Hubbert C, Guardiola A, Shao R, Kawaguchi Y, Ito A, Nixon A, et al HDAC6 is a microtubule-associated Mis18α. Nature 2002;417:455-458).

Meanwhile, miRNA is a type of endogenous small RNA of 20 to 25 nucleotides in length that exists within cells and is derived from DNA that does not synthesize proteins to be generated from a hairpin-shaped transcript. miRNA binds to a complementary sequence of the 3'-UTR of target mRNA and induces translational inhibition or destabilization of the mRNA to ultimately serve as a repressor that inhibits protein synthesis of the target mRNA. It is known that one miRNA targets a plurality of mRNAs, and mRNAs may also be regulated by a plurality of miRNAs.

Meanwhile, macrophages phagocytose disease cells (cancer cells) through phagocytosis, which occurs by mediating the binding of Fc fragments of antibodies to Fc receptors on the membrane surface of macrophages. However, tumors may escape from the attack of immune cells, including macrophages, by overturning normal immune regulatory mechanisms. One of these mechanisms is CD47, a protein expressed in normal cells. CD47 interacts with a macrophage receptor called signal-regulatory protein α (SIRPα), which leads to the signaling of "don't eat me" (phagocytosis blocking) to macrophages so as to leave normal cells. Likewise, CD47 expression by cancer cells has resistance to macrophages even when the cancer cells bind to antibodies. Accordingly, although a large number of macrophages approach tumors, the macrophages cannot act on cancer cells unless the "phagocytosis blocking" signal is turned off. One treatment strategy thereof is to block the "phagocytosis blocking" signal using a monoclonal antibody against CD47.

Therefore, it was confirmed that the present invention can be used as a pharmaceutical composition for preventing or treating liver cancer by modifying RT-LET7, an expression inhibitor of Let-7i-5p, to increase the effect compared to conventional RT-LET7. In addition, the modified RT-LET7 increases TSP1 by inhibiting Let-7i-5p in liver cancer cells, and the increased TSP1 binds to CD47 to block interaction with SIRPα of macrophages. Therefore, it was confirmed that the modified RT-LET7 increased the immune activity of macrophages against cancer cells by blocking the binding of CD47 on liver cancer cells and SIRPα on macrophages and significantly increased the phagocytosis activity of macrophages through a mechanism that exhibited anticancer effects, and thus can be used as a checkpoint inhibitor for treatment of CD47-positive liver cancer, and then the present invention was completed.

### [Disclosure]

### [Technical Problem]

An object of the present invention is to provide a nucleic acid molecule targeting Let-7i-5p.

Another object of the present invention is to provide a nucleic acid molecule with which a liver cell-targeted moiety is combined.

Yet another object of the present invention is to provide a pharmaceutical composition for preventing or treating liver cancer including the nucleic acid molecule as an active ingredient.

Yet another object of the present invention is to provide a checkpoint inhibitor for treating CD47-positive liver cancer including the nucleic acid molecule as an active ingredient.

Yet another object of the present invention is to provide a method for preventing or treating liver cancer including administering the pharmaceutical composition to a subject.

Yet another object of the present invention is to provide a nucleic acid molecule targeting Let-7i-5p, in which in the nucleic acid molecule represented by SEQ ID NO: 1, a nucleotide sequence is modified by 2'-O-methoxyethylation, a part of the backbone is modified with phosphorothioate, and a liver cell-targeted moiety is combined.

### [Technical Solution]

An aspect of the present invention provides a nucleic acid molecule targeting Let-7i-5p, in which the nucleic acid molecule is represented by a base sequence of SEQ ID NO: 1, the nucleic acid molecule is a nucleic acid in which a nucleotide sequence is modified by 2'-O-methoxyethylation, and a part or the entire backbone of the nucleic acid molecule is modified with phosphorothioate.

Another aspect of the present invention provides a nucleic acid molecule targeting Let-7i-5p, in which the nucleic acid molecule is represented by a base sequence of SEQ ID NO: 1, the nucleic acid molecule is a nucleic acid in which a nucleotide sequence is modified by 2'-O-methoxyethylation, a part or the entire backbone of the nucleic acid molecule is modified with phosphorothioate, and the nucleic acid molecule is combined with a liver cell-targeted moiety.

Yet another aspect of the present invention provides a pharmaceutical composition for preventing or treating liver cancer including modified RT-LET7 as an active ingredient.

Yet another aspect of the present invention provides a pharmaceutical composition for preventing or treating liver cancer including modified RT-LET7 combined with a liver cell-targeted moiety as an active ingredient

Yet another aspect of the present invention provides a checkpoint inhibitor pharmaceutical composition for treating CD47-positive liver cancer including modified RT-LET7 as an active ingredient.

Yet another aspect of the present invention provides a checkpoint inhibitor for treating CD47-positive liver cancer including modified RT-LET7 combined with a liver cell-targeted moiety as an active ingredient

Yet another aspect of the present invention provides a method for preventing or treating liver cancer including administering the pharmaceutical composition to a subject.

### [Advantageous Effects]

According to the present invention, compared to a conventional RT-LET7, which is antisense microRNA (AS-miRNA) for inhibiting Let-7i-5p, the modified RT-LET7 or the modified RT-LET7 combined with the liver cell-target moiety increases the inhibition of Let-7i-5p expression, increases the inhibition of liver cancer cell growth, increases TSP1 expression, and increases phagocytosis activity of macrophages. In addition, the liver cell-targeted moiety is combined with the modified RT-LET7 so as to improve delivery to liver cancer cells and remarkably improve the effectiveness of liver cancer treatment, and thus the modified RT-LET7 can be effectively used in a pharmaceutical composition for prevention or treatment of liver cancer, or as a checkpoint inhibitor for prevention or treatment of CD47-positive liver cancer.

### [Description of Drawings]

FIG. 1 is a diagram illustrating various modified structures (FIG. 1A) and inhibition efficiency (FIG. 1B) of RT-LET7, an expression inhibitor of Let-7i-5p, in an exemplary embodiment of the present invention (basic type: RT-LET7, modified type: RT-LET7-2, RT-LET7-4, RT-LET7-6 and RT-LET7-8).
FIG. 2 is a diagram confirming an effect of RT-LET7-8, modified RT-LET7 of the present invention, on significantly and continuously inhibiting Let-7i-5p in HCC cell lines in an exemplary embodiment of the present invention (FIG. 2A; SNU-387, FIG. 2B; SNU-368, FIG. 2C; SNU-423).
FIG. 3 is a diagram confirming the growth inhibition of HCC cell lines and increased activity of macrophages by RT-LET7-8, modified RT-LET7 of the present invention, in an exemplary embodiment of the present invention (FIG. 3A; tumorigenesis characteristics of Let-7i-5p through MTT and cell survival analysis, FIG. 3B; TSP1 expression change through Western blot analysis after treatment with basic RT-LET7 and modified RT-LET7-8, FIG. 3C; phagocytosis activity of macrophages in HCC cells treated with basic RT-LET7 and modified RT-LET7-8).
FIG. 4 is a diagram confirming the inhibition efficiency of Let-7i-5p expression and an effect of continuously inhibiting Let-7i-5p in HCC cells by RT-LET7-8 combined with GalNAc.
FIG. 5 is a diagram confirming the inhibition efficiency of Let-7i-5p expression and an effect of continuously inhibiting Let-7i-5p in HCC cells by RT-LET7-8 combined with Gal-LNP, in an exemplary embodiment of the present invention.
FIG. 6 is a diagram confirming the growth inhibition of HCC cell lines and increased activity of macrophages by RT-LET7-8 combined with GalNAc, in an exemplary embodiment of the present invention (FIG. 6A; tumorigenesis characteristics of Let-7i-5p through MTT and cell survival analysis, FIG. 6B; TSP1 expression change through Western blot analysis after treatment with RT-LET7-8 combined with GalNAc, FIG. 6C; phagocytosis activity of macrophages in HCC cells treated with RT-LET7-8 combined with GalNAc).
FIG. 7 is a diagram confirming the growth inhibition of HCC cell lines and increased activity of macrophages by RT-LET7-8 combined with Gal-LNP, in an exemplary embodiment of the present invention (FIG. 6A; tumorigenesis characteristics of Let-7i-5p through MTT and cell survival analysis, FIG. 6B; TSP1 expression change through Western blot analysis after treatment with RT-LET7-8 combined with Gal-LNP, FIG. 6C; phagocytosis activity of macrophages in HCC cells treated with RT-LET7-8 combined with Gal-LNP).
FIG. 8 is a schematic diagram briefly illustrating the binding of liver cell-targeted delivery systems GalNAc and Gal-LNP in an exemplary embodiment of the present invention (FIG. 8A; Gal-LNP binding, FIG. 8B; Old-GalNAc binding, FIG. 8C; D-GalNAc binding).
FIG. 9 is a diagram confirming the inhibition efficiency of Let-7i-5p expression and an effect of continuously inhibiting Let-7i-5p in HCC cells by RT-LET7-8 combined with GalNAc (D-GalNAc) having a different structure, in an exemplary embodiment of the present invention.
FIG. 10 is a diagram confirming the growth inhibition of HCC cell lines by RT-LET7-8 combined with GalNAc (D-GalNAc) having a different structure, in an exemplary embodiment of the present invention.
FIG. 11 is a diagram confirming changes in TSP1 expression of HCC cell lines and increased activity of macrophages by RT-LET7-8 combined with GalNAc (D-GalNAc) having a different structure, in an exemplary embodiment of the present invention.

### [Best mode of the Invention]

Hereinafter, exemplary embodiments of the present invention will be described in detail with reference to the accompanying drawings. However, the following exemplary embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present disclosure are possible within the description of claims to be described below and the equivalent scope interpreted therefrom.

Terminologies used herein are terminologies used to properly express preferred exemplary embodiments of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

In an aspect, the present invention relates to a nucleic acid molecule targeting Let-7i-5p, in which the nucleic acid molecule is represented by SEQ ID NO: 1.

In an exemplary embodiment, in the "nucleic acid molecule" of the present invention, a nucleotide sequence may be modified by 2'-O-methoxyethylation.

In an exemplary embodiment, in the "nucleic acid molecule" of the present invention, a part or the entire backbone thereof may be modified with phosphorothioate, and preferably, among nucleotides in SEQ ID NO: 1, backbones of first to fourth nucleotides from a 5' terminus and first to fourth nucleotides from a 3' terminus may be modified with phosphorothioate, but it is not limited thereto.

In an exemplary embodiment, the "nucleic acid molecule" of the present invention may be combined with a liver cell-targeted moiety.

In addition, the liver cell-targeted moiety may be used without limitation as long as it is known as a liver tissue-targeted drug delivery system, and includes, for example, N-acetylgalactosamine (GalNAc), galactosyl lipidoid nanoparticle (Gal-LNP), lipid-siRNA, antibody-siRNA, peptide-ASO, stable nucleic acid lipid particle, exosome, spherical nucleic acid, DNA cage, etc. (Nat Rev Drug Discov. 2020 Oct;19(10):673-694.).

In addition, the galactosyl lipidoid nanoparticle (Gal-LNP) may consist of cholesterol, DSPC, C16-PET2000-ceramide, and α-galactosyl ceramide.

In addition, the N-acetylgalactosamine (GalNAc) may be represented by Chemical Formula 1 or 2, and preferably Chemical Formula 2, but is not limited thereto.

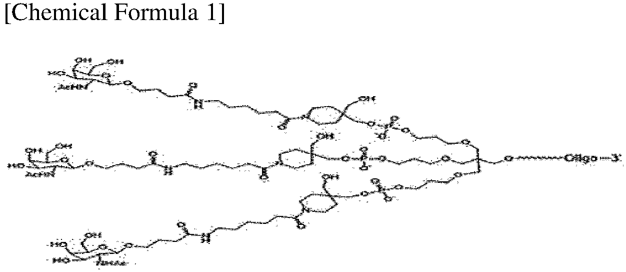

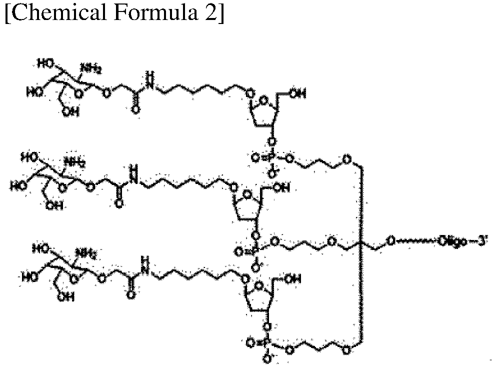

In an aspect, the present invention relates to a pharmaceutical composition for preventing or treating liver cancer including the nucleic acid molecule as an active ingredient.

In an exemplary embodiment, the "composition" of the present invention may inhibit the expression of Let-7i-5p.

In an exemplary embodiment, the "composition" of the present invention may inhibit liver cancer cell growth.

In an exemplary embodiment, the "composition" of the present invention may increase thrombospondin-1 (TSP1) expression.

In an exemplary embodiment, the "composition" of the present invention may increase phagocytosis activity of macrophages.

In an aspect, the present invention relates to a checkpoint inhibitor for treating CD47-positive liver cancer including the nucleic acid molecule as an active ingredient.

In an exemplary embodiment, the "TSP1" of the present invention may capture a CD47 receptor to hinder the interaction of CD47-SIRPα.

In an exemplary embodiment, the "nucleic acid molecule" of the present invention may regulate a let-7i-p-TSP1 signaling axis, and converts the CD47-SIRPα interaction between macrophages and HCC to CD47-TSP1 interaction, thereby reactivating phagocytosis of macrophages on HCC cells.

In an exemplary embodiment, the "liver cancer" of the present invention may be liver cancer with high Let-7i-5p expression, may be hepatocellular carcinoma, may be hepatocellular carcinoma of stage I, II, III, IVA or IVB phase, and more preferably hepatocellular carcinoma of stage III to IV phase, which is more difficult to be treated than the early stage.

In an exemplary embodiment, the "liver cancer" of the present invention may be liver cancer with low TSP1 expression, may be hepatocellular carcinoma, may be hepatocellular carcinoma of stage I, II, III, IVA or IVB phase, and more preferably hepatocellular carcinoma of stage III to IV phase, which is more difficult to be treated than the early stage.

As used herein, the term "expression inhibition" means causing a decrease in expression (to mRNA) or translation (to protein) of a target gene, and preferably means that the expression of the target gene becomes undetectable or exists at an insignificant level thereby.

As used herein, the term "prevention" refers to all actions that inhibit or delay the occurrence, development, and recurrence of liver cancer by administration of the composition according to the present invention.

As used herein, the term "treatment" means all actions that improve or beneficially change the symptoms of liver cancer and its complications by administration of the composition according to the present invention. Those skilled in the art to which the present invention pertains will be able to determine the degree of improvement, enhancement and treatment by knowing the exact criteria of a disease for which the composition of the present invention is effective by referring to data presented by the Korean Academy of Medical Sciences, etc.

In an exemplary embodiment, the pharmaceutical composition may be one or more formulations selected from the group including oral formulations, external formulations, suppositories, sterile injection solutions and sprays.

The composition of the present invention may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using as a suitable method in the art or a method disclosed in Remington's Pharmaceutical Science (Mack Publishing Company, Easton PA, 18th, 1990).

The composition of the present invention may further contain one or more active ingredients exhibiting the same or similar functions. The composition of the present invention includes 0.0001 to 10 wt%, preferably 0.001 to 1 wt% of the protein, based on the total weight of the composition.

The pharmaceutical composition of the present invention may further include a pharmaceutically acceptable additive. At this time, the pharmaceutically acceptable additive may use starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present invention is preferably included in an amount of 0.1 part by weight to 90 parts by weight based on the composition, but is not limited thereto.

The composition of the present invention may be administered parenterally (for example, intravenously, subcutaneously, intraperitoneally, or topically) or orally depending on a desired method, and most preferably orally administered. The dose range thereof varies according to the weight, age, sex, health condition, diet, administration time, administration method, and excretion rate of a subject, the severity of a disease, etc.

Liquid formulations for oral administration of the composition of the present invention correspond to suspensions, internal solutions, emulsions, syrups, etc., and may include various excipients, such as wetting agents, sweeteners, fragrances, preservatives, and the like in addition to water and liquid paraffin, which are commonly used simple diluents. Formulations for parenteral administration include sterilized aqueous solutions, non-aqueous solvents, suspensions, emulsions, lyophilized agents, suppositories, and the like.

In an aspect, the present invention relates to a method for treating liver cancer including administering the pharmaceutical composition or the checkpoint inhibitor to a subject.

As used herein, the term "subject" means a subject in need of a method for preventing, controlling or treating a disease, and may be used without limitation, such as humans, dogs, monkeys, cats, rodents, such as mice and genetically engineered mice. More specifically, the subject refers to mammals such as humans or non-human primates, mice, rats, dogs, cats, horses, and cows.

The pharmaceutical composition of the present invention may be administered in a therapeutically effective dose or a pharmaceutically effective dose.

As used herein, the term "therapeutically effective dose" means an amount of a pharmaceutically acceptable salt of the composition effective for preventing or treating a target disease, and the therapeutically effective dose of the composition of the present invention may vary depending on many factors, such as a method of administration, a target site, the condition of a patient, and the like. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective dose. As used herein, the term "pharmaceutically effective dose" refers to an amount enough to treat the disease at a reasonable benefit/risk ratio applicable to medical treatment, and does not cause side effects. The effective dose level may be determined according to factors including the health condition of a subject, the type and severity of infectious diseases, the activity of a drug, the sensitivity to a drug, an administration method, a time of administration, a route of administration, an excretion rate, duration of treatment, and drugs used in combination or simultaneously, and other factors well-known in the medical field. The composition of the present invention may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

### [Modes of the Invention]

Hereinafter, the present invention will be described in more detail through Examples. However, these Examples are more specifically illustrative the present invention, and the scope of the present invention is not limited to these Examples.

### <Example 1> Preparation of modified RT-LET7 of conventional RT-LET7

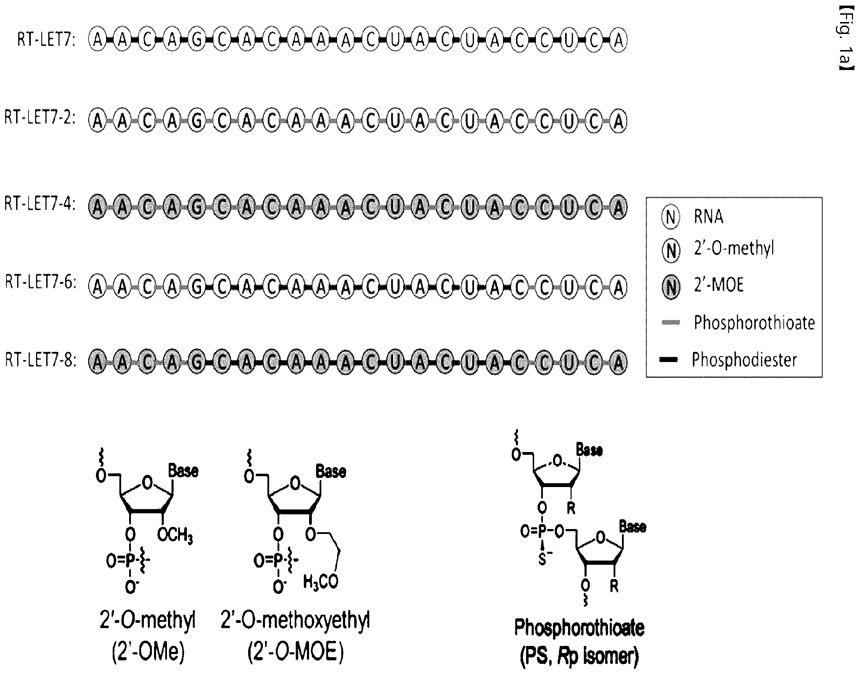

In RT-LET7 (SEQ ID NO: 1: AACAGCACAAACUACUACCUCA), an inhibitor of Let-7i-5p, one cycle of a four-step process was performed, and RNA oligo 1 mer was processed sequentially from the 3' end to the 5' end through processes of Deblocking -> Coupling -> Oxidation -> Capping. At this time, modified bases are used, but according to Nat Rev Drug Discov. 2020 Oct;19(10):673-694., modifications 2'-O-methyl and 2'-MOE of 2'-Ribose contribute to increasing resistance from nucleases compared to unmodified RNA, and increasing stability within the cytoplasm. In addition, the modifications serve to increase the half-life in living tissue, thereby ultimately increasing the effect of a drug. Moreover, the modifications are strongly attached to complementary RNA to help target genes to be degraded more effectively by RNase. Additionally, when phosphorothioate was substituted between RNA bases, the modifications do not affect RNase activity and efficiently binds to the target RNA. In addition, the modifications bind to proteins such as albumin in cells and the body to be protected from nuclease, thereby preventing the drug from being excreted through urine, increasing the duration of the drug in the body and improving the drug's effect. For this reason, Ribose and phosphorothioate modifications were performed. Modified RNA bases 2'-OMe rA Phosphoramidite (Glen Research, Sterling, VA, cat. 10-3100), 2'-OMe rC Phosphoramidite (Glen Research, cat. 10-3115), 2'-OMe rG Phosphoramidite (Glen Research, cat. 10-3120), 2'-OMe rU Phosphoramidite (Glen Research, cat. 10-3130), 2'-MOE rA Phosphoramidite (Glen Research, cat. 10-3200), 2'-MOE rC Phosphoramidite (Glen Research, cat. 10-3211), 2'-MOE rG Phosphoramidite (Glen Research, cat. 10-3220), and 2'-MOE rU Phosphoramidite (Glen Research, cat. 10-3231) were synthesized. During the synthesis process, parts requiring substitution with phosphorothioate were synthesized using a Sulfurizing Reagent II (Glen Research, cat. 40-4037-10) (Bioneer synthesis). At this time, RNA oligo without any modification was named RT-LET7, RNA oligo having all bases modified with 2'-O-methyl and modified with phosphorothioate was named RT-LET7-2, and bases modified at four positions at the 5 and 3' ends were named RT-LET7-6. In addition, RNA oligo having all bases modified with 2'-MOE and modified with phosphorothioate was named RT-LET7-4, and bases modified at four positions at the 5 and 3' ends were named RT-LET7-8 (FIG. 1A).

### <Example 2> Confirmation of inhibition of Let-7i-5p expression by treatment with modified RT-LET7

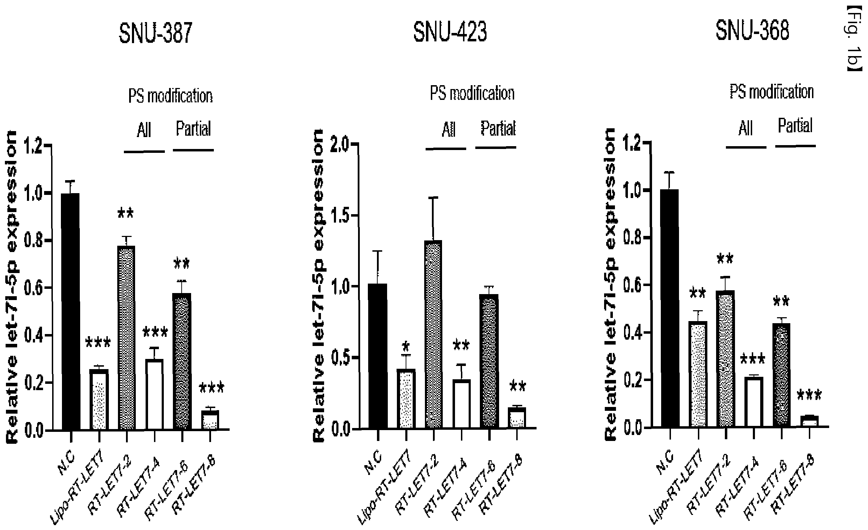

Total RNA was isolated from HCC cell lines SNU-387, SNU-368, and SNU-423 transfected with the modified RT-LET7 as shown in FIG. 1A using a TRIzol reagent (Invitrogen, Carlsbad, CA), and then cDNA specific to the two miRNAs was synthesized using a micscipt II RT kit (Qiagen, Manchester, UK). qRT-PCR was performed with a SensiFASTTM SYBR NoROX Kit (Bioline, London, UK). As a result of performing qRT-PCR analysis of Let-7i-5p in HCC cell lines SNU-387, SNU-368, and SNU-423, it was confirmed that when all bases were substituted with 2'-MOE (RT-LET7-4 and RT-LET7-8), it was more effective in inhibiting the expression of -7i-5p than when all the bases were substituted with 2'-O-methyl (RT-LET7-2 and RT-LET7-6) (FIG. 1B).

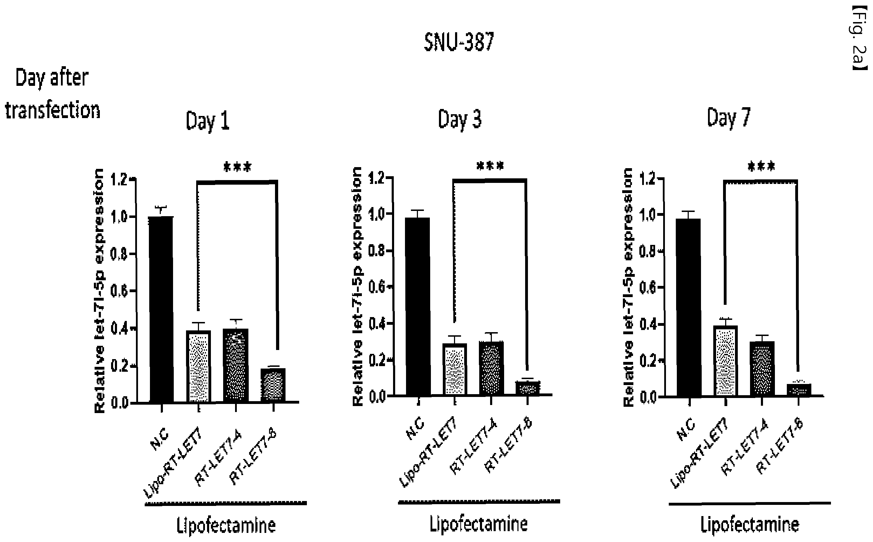

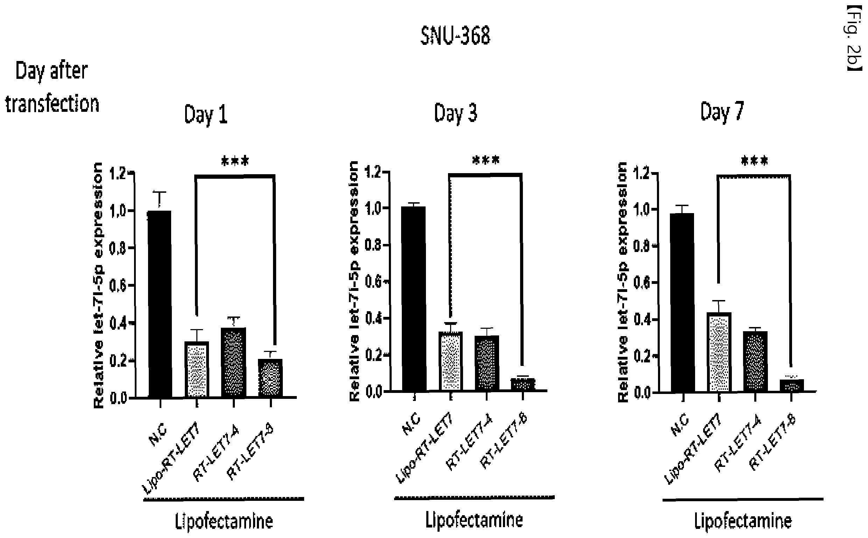

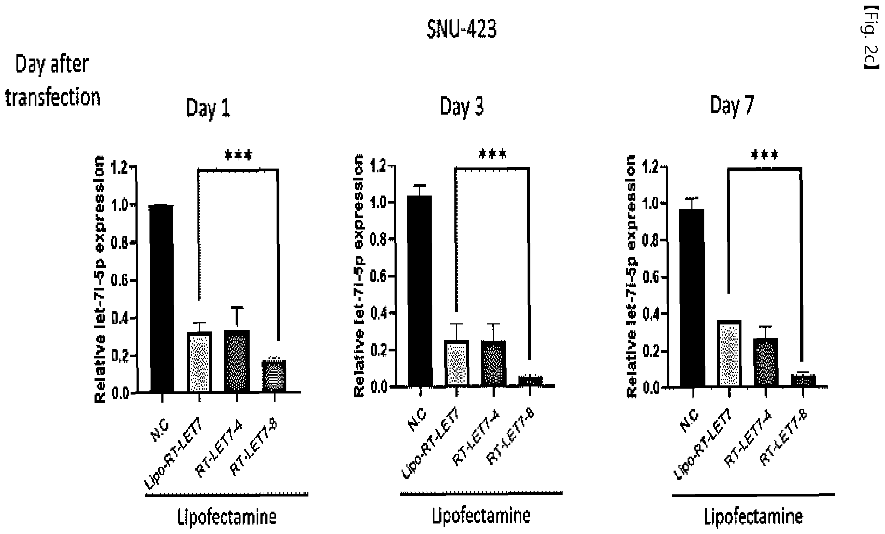

In addition, when RT-LET7 was modified with 2'-MOE, HCC cell lines SNU-387, SNU-368, and SNU-423 were cultured up to 7 days after transfection, and then RNA was extracted, and the degree of inhibition of Let-7i-5p expression was compared. At this time, it was confirmed that when RT-LET7 was modified with 2'-MOE and then substituted with phosphorothioate, the efficiency of the inhibition of Let-7i-5p expression was significantly increased compared to RT-LET7 without any modification (FIG. 2).

### <Example 3> Confirmation of tumorigenesis characteristics of Let-7i-5p through MTT and cell survival analysis

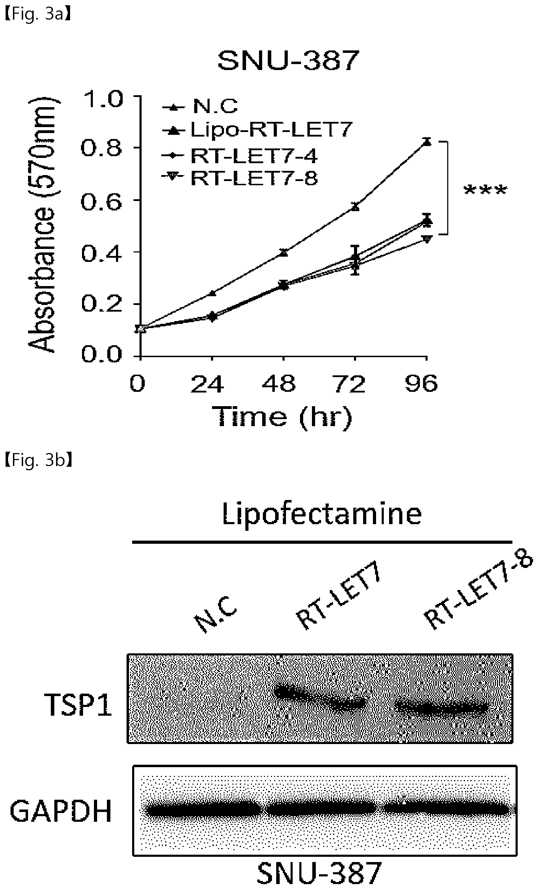

To confirm the inhibition of in vitro tumorigenesis upon RT-LET7 treatment in liver cancer, MTT assay was performed using RT-LET7. Specifically, for MTT assay, SNU-387 cells were dispensed in a 12-well plate, transfected with RT-LET7, RT-LET7-4, and RT-LET7-8, and then incubated with 0.5 mg/ml of an MTT [3-(4,5-dimethylthiazol-2-yl)-2,5-diphenyltetrazolium bromide] solution (Sigma) for 1 hour. Thereafter, absorbance was measured using a SYNERGY H1 Multilabel plate reader (Bio-Tek, Winooski, VT). As a result, it was confirmed that in an experimental group treated with RT-LET7-8 as modified RT-LET7, tumor cell growth was best inhibited (FIG. 3A).

### <Example 4> Confirmation of regulation of macrophage phagocytosis by modified RT-LET7

To determine whether Let-7i-5p regulated the expression of TSP1, which was involved in the regulation of macrophage phagocytosis, expression changes were confirmed through Western blot analysis after treatment with RT-LET7 and modified RT-LET7-8. As a result, it was confirmed that the expression of TSP1 was increased when RT-LET7 and RT-LET7-8 were treated, respectively (FIG. 3B).

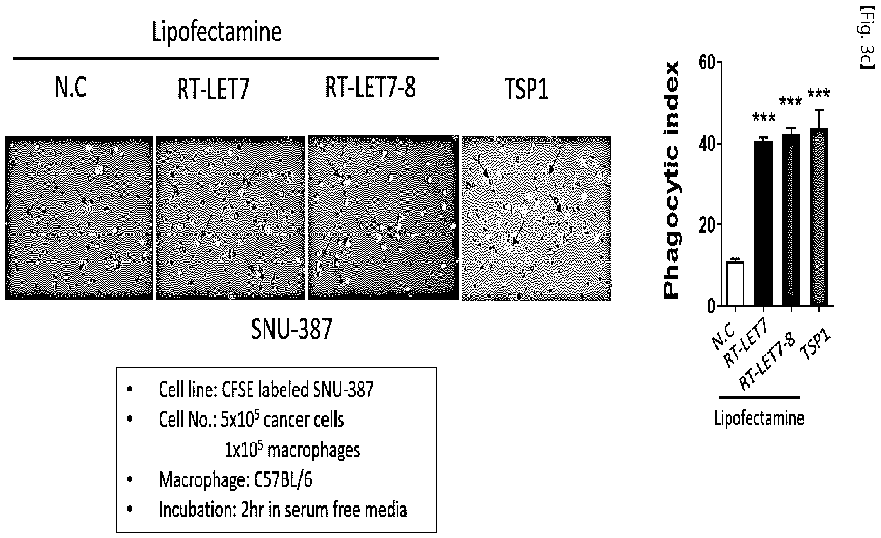

Based on these results, in the interaction between CD47 and signal regulatory protein α (SIRPα) in a Let-7i-5p-TSP1 network, TSP1 captured the CD47 receptor to hinder the interaction of CD47-SIRPα, and thus to determine whether macrophages were able to phagocytose HCC, in vitro phagocytosis analysis was performed. Specifically, each HCC cell line, SNU-387, was made into a single cell suspension and then labeled with CFSE (abcam, Cambridge, UK). Thereafter, peritoneal macrophages were obtained from C57BL/6 mice, co-cultured with SNU-387 for 2 hours, and then treated with RT-LET7 and modified RT-LET7-8, respectively, and then compared with HCC cells directly treated with TSP1 recombinant protein as a positive control group. The phagocytic index was calculated by dividing the number of macrophages capturing tumor cells by the total number of macrophages. As a result, it was confirmed that the macrophage phagocytosis activity was significantly increased in all HCC cells treated with RT-LET7 and modified RT-LET7-8, and the macrophage phagocytosis activity in modified RT-LET7-8 was further increased compared to RT-LET7 (FIG. 3C).

As a result, it may be seen that RT-LET7-8 as modified RT-LET7 may regulate the let-7i-p-TSP1 signaling axis, and the CD47-SIRPα interaction between macrophages and HCC was converted to a CD47-TSP1 interaction to re-activate the phagocytosis of macrophages against HCC cells.

### <Example 5> Verification of efficiency of RT-LET7-8 combined with liver cell-targeted moiety

### 5-1. Confirmation of inhibition of Let-7i-5p expression by RT-LET7-8 using delivery system

To confirm an effect of modified RT-LET7-8 on inhibiting the expression of Let-7i-5p using the delivery system, nucleic acid molecules in which RT-LET7-8 was combined with N-Acetylgalactosamine (GalNAc) or Galactosyl lipidoid nanoparticle (Gal-LNP), known as a drug delivery system, were delivered to positive cells (Hep3B) and negative cells (SNU-449) to the expression of an asialoglycoprotein receptor (ASGPR).

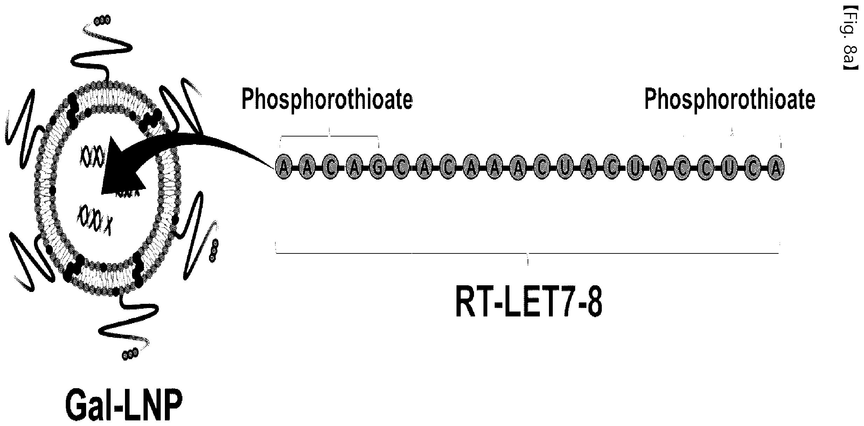

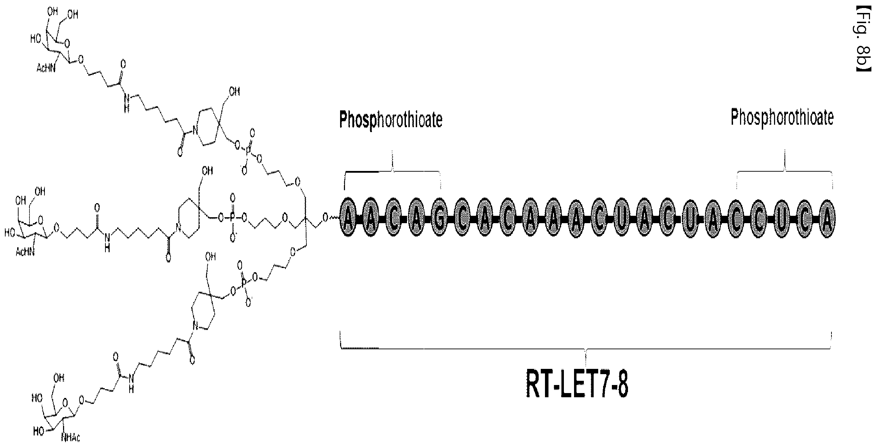

Specifically, Gal-LNP-RT-LET7-8 was mixed with C12-SPM synthesized by combining alkyl epoxide (Sigma-Aldrich) and polyamine (Sigma-Aldrich), Distearoylphosphatidylcholine (DSPC) (Sigma-Aldrich), cholesterol (Sigma-Aldrich), C16-PEG2000-ceramide (Avanti Polar Lipids) and α-galactosyl ceramide (Avanti Polar Lipids) at a ratio of 50 : 10 : 38.5 : 0.75 : 0.75, respectively, to prepare Gal-LNP. Thereafter, a RT-LET7-8 solution (10 mg/mL) was mixed with 10 mM citrate buffer (pH 3), mixed with Gal-LNP and RT-LET7-8 in a ratio of 7 : 1, and incubated at 37°C for 30 minutes, and thus RT-LET7-8 was loaded onto Gal-LNP. Thereafter, the loaded RT-LET7-8 was dialyzed with PBS (Sigma-Aldrich, cat. P5368) for 75 minutes to remove ethanol and RT-LET7-8 that was not loaded with Gal-LNP (see FIG. 8A). Subsequently, GalNAc-RT-LET7-8 was synthesized by conjugating Trebler phosphoramidite (GLEN Research) to the 5' end of modified RT-LET7-8 to modify RT-LET7-8 and then conjugating N-acetylgalactosamine (GLEN Research) (see FIG. 8B). Thereafter, the expression level of Let-7i-5p in each cell was confirmed in the same manner as in Example 2.

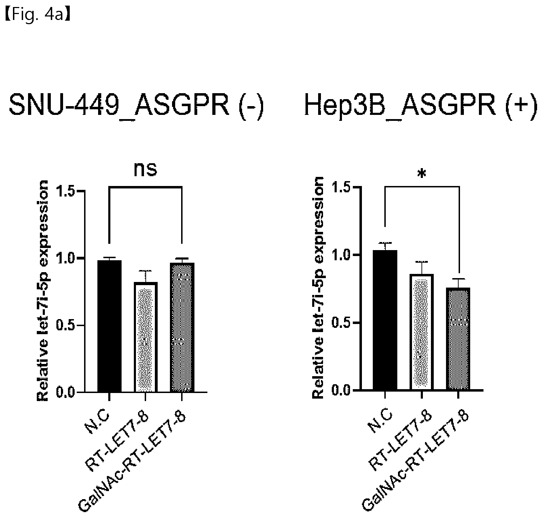

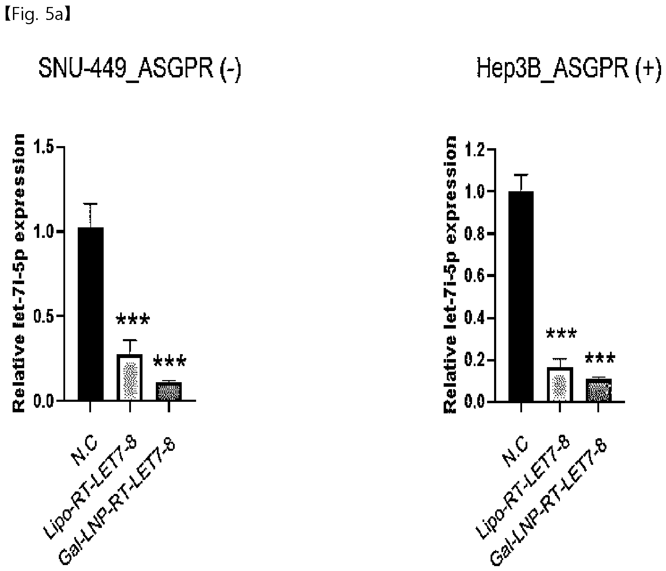

As a result, in the case of RT-LET7-8 combined with GalNAc, the inhibitory effect of Let-7i-5p expression was significantly increased compared to the conventional RT-LET7-8 in Hep3B cells, which were ASGPR positive cells, but the inhibitory effect of Let-7i-5p expression was not increased in SNU-449 cells, which were ASGPR negative cells (FIG. 4A). On the other hand, in the case of RT-LET7-8 combined with Gal-LNP, the inhibitory effect of Let-7i-5p expression was significantly increased regardless of the presence of ASGPR (FIG. 5A). These results showed that GalNAc was a delivery system having a property of binding to the ASGPR receptor on the surface of liver cells, which was effective only on ASGPR positive cells. However, Gal-LNP had a structure similar to the cell membrane, so that it is expected to have an effect regardless of ASGPR.

In addition, in RT-LET7-8 (Gal-LNP-RT-LET7-8) combined with Gal-LNP, the Let-7i-5p expression was significantly reduced compared to RT-LET7-8 (Lipo-RT-LET7-8) combined with lipofectamine. The lipofectamine consisted of simple phospholipids, and thus, compared to Gal-LNP consisting of cholesterol, DSPC, C 16-PET2000-ceramide, and α-galactosyl ceramide, the delivery efficiency to the liver and stability in vivo were significantly lowered.

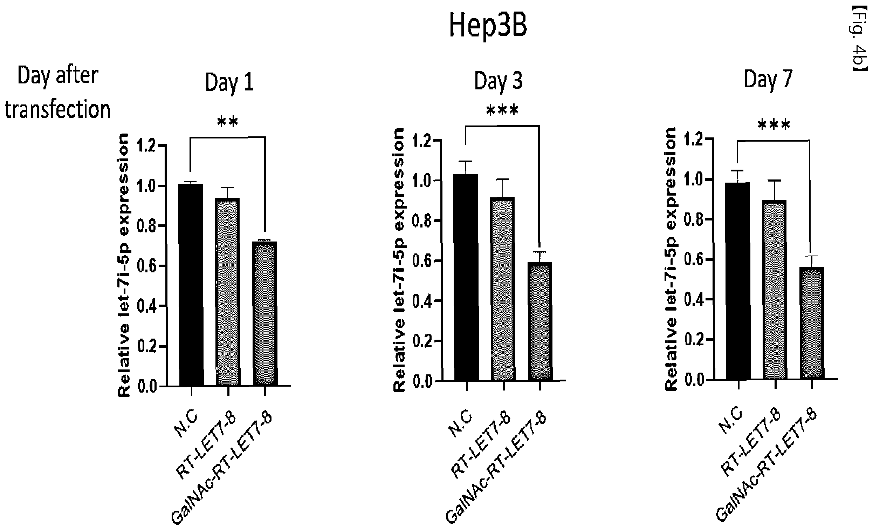

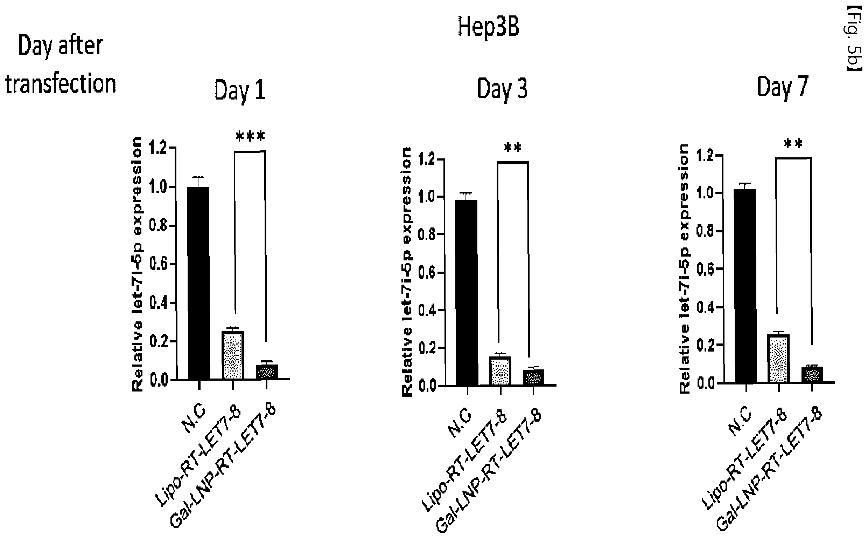

Subsequently, the efficiency retention of RT-LET7-8 combined with the liver cell-targeted moiety GalNAc or Gal-LNP for 7 days was verified in Hep3B cells. As a result, it was shown that the inhibitory effect of Let-7i-5p expression was maintained until 7 days, and the Let-7i-5p expression was stably inhibited compared to Lipo-RT-LET7-8 (FIGS. 4B and 5B).

### 5-2. Confirmation of inhibition of tumorigenesis in liver cancer by RT-LET7-8 using delivery system

To confirm the inhibition of in vitro tumorigenesis when treated with RT-LET7-8 combined with a liver cell-targeted moiety in liver cancer, MTT assay was performed using the method of Example 3.

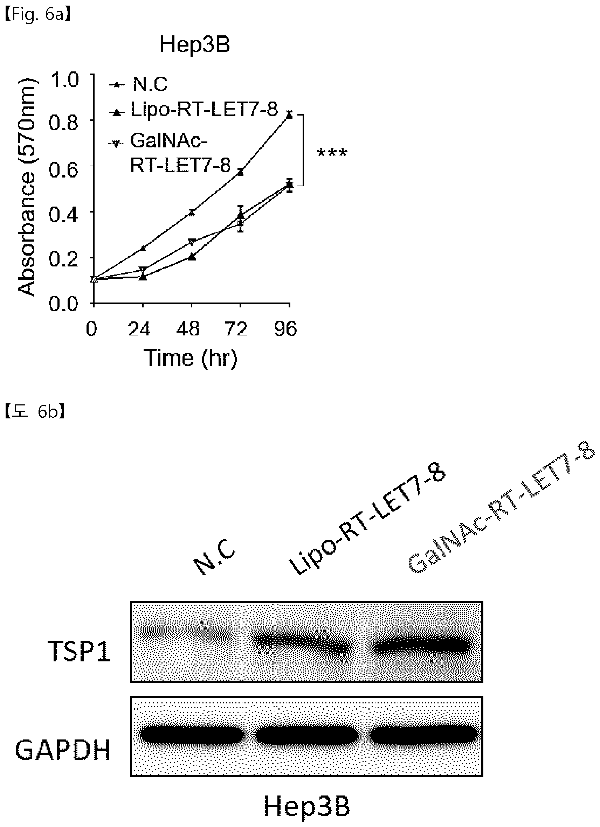

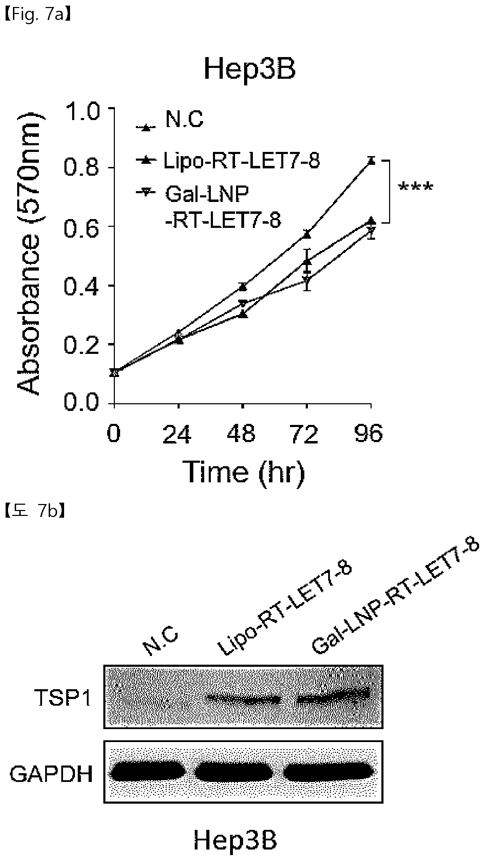

As a result, it was shown that in the case of GalNAc-RT-LET7-8 and Gal-LNP-RT-LET7-8 combined with the liver cell-targeted moiety, compared to a control group, the tumorigenesis of liver cancer cells was effectively inhibited, and even compared to Lipo-RT-LET7-8, high tumorigenesis inhibition was shown after 72 hours (FIGS. 6A and 7A).

### 5-3. Confirmation of regulation of macrophage phagocytosis by RT-LET7-8 using delivery system

To confirm changes in the expression of TSP1, a target protein oflet-7i-5p, by lowering the expression of let-7i-5p, target microRNA of RT-LET7, modified RT-LET7-8 and RT-LET7-8 combined with the liver cell-targeted moiety were treated and then expression changes were confirmed through Western blot analysis.

As a result, it was confirmed that in the case of RT-LET7-8 combined with the liver cell-targeted moiety, the expression of TSP1 was increased compared to modified RT-LET7-8 that was not combined with the liver cell-targeted moiety (FIGS. 6A and 7B).

Next, in order to confirm whether macrophages were able to phagocytose HCC, in vitro phagocytosis assay was performed using the method of Example 4.

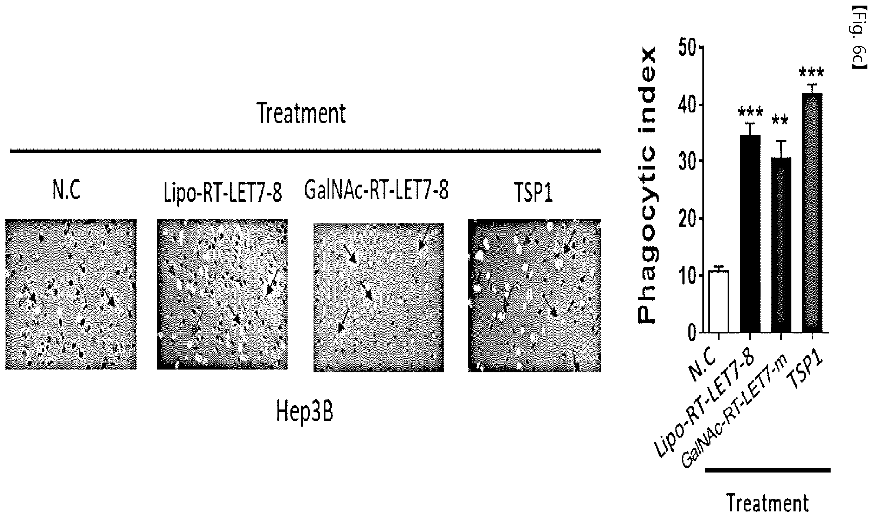

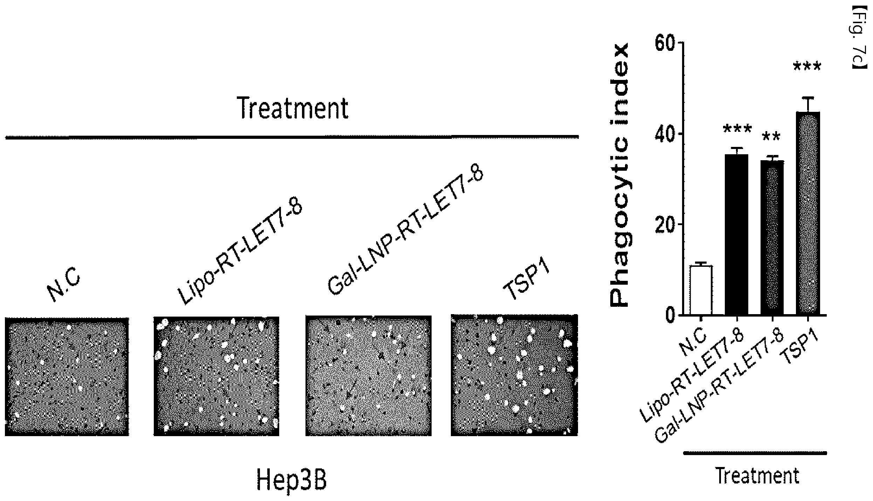

As a result, it was confirmed that macrophage phagocytosis activity was further increased in GalNAc-RT-LET7-8 and Gal-LNP-RT-LET7-8 compared to modified RT-LET7-8 (FIGS. 6C and 7C).

### <Example 6> Verification of efficiency of RT-LET7-8 combined with D-GaINAc

Through Example 5, it was confirmed that when combined with modified RT-LET7-8, GalNAc had higher delivery efficiency to liver and in vivo stability compared to Gal-LNP.

Accordingly, in order to search for the optimal GalNAc capable of significantly improving the efficacy of RT-LET7-8 by combining modified RT-LET7-8, RT-LET7-8 was prepared in which GalNAc (D-GalNAc) having a different structure was combined with the 5' end, and the efficiency of the modified RT-LET7-8 combined with GalNAc was compared through the following experiment.

### 6-1. Confirmation of inhibition of Let-7i-5p expression by D-GalNAc-RT-LET7-8

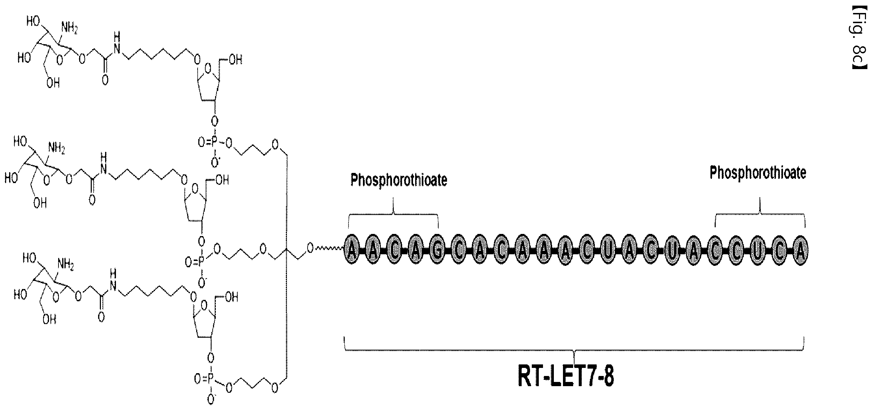

RT-LET7-8 was prepared by combining GalNAc (Old-GalNAc) used in Example 5 and new GalNAc, D-GalNAc, and the two types of Old-GalNAc-RT-LET7-8 (see FIG. 8B) and D-GalNAc-RT-LET7-8 (see FIG. 8C) were each delivered to Hep3B cells and the Let-7i-5p expression level was confirmed in each cell in the same manner as in Example 2.

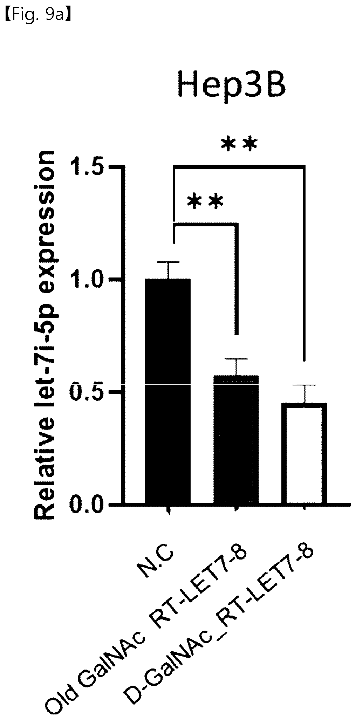

As a result, it was confirmed that in the case of RT-LET7-8 combined with D-GalNac, the inhibitory effect of Let-7i-5p expression was significantly increased compared to the conventional Old-GalNAc-RT-LET7-8 in Hep3B cells, which were ASGPR positive cells (FIG. 9A).

Subsequently, efficiency retention verification of Old-GalNAc-RT-LET7-8 and D-GalNAc-RT-LET7-8 for 14 days was performed in Hep3B cells, and the efficiency retention verification test was performed by varying temperature conditions (4°C/room temperature).

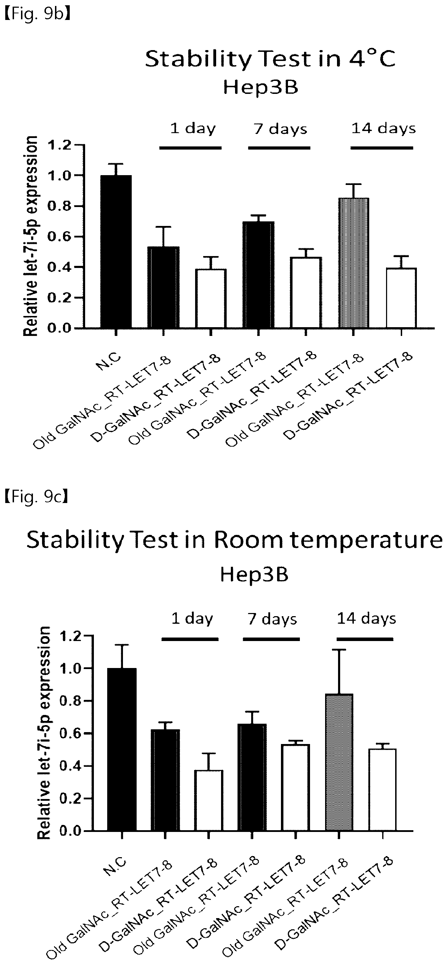

As a result, in the case of Old-GalNAc-RT-LET7-8, the inhibitory effect of Let-7i-5p expression was maintained until 7 days, but after 7 days, the Let-7i-5p expression was recovered to a level similar to that of the control group. However, in the case of GalNAc-RT-LET7-8, it was shown to stably inhibit the Let-7i-5p expression up to 14 days (FIGS. 9B and 9C).

### 6-2. Confirmation of inhibition of tumorigenesis in liver cancer by GalNAc-RT-LET7-8

To confirm the inhibition of in vitro tumorigenesis in liver cancer when treated with RT-LET7-8 combined with GalNAc (Old-GalNAc) and the new GalNAc, D-GaINAc, Old-GalNAc-RT-LET7-8 and D-GalNAc-RT-LET7-8 were delivered to Hep3B cells, respectively, and then MTT assay and BrdU assay were performed.

The MTT assay was performed by the method described in Example 3, and the BrdU assay was performed using a BrdU cell proliferation assay kit (Millipore) according to the manufacturer's protocol.

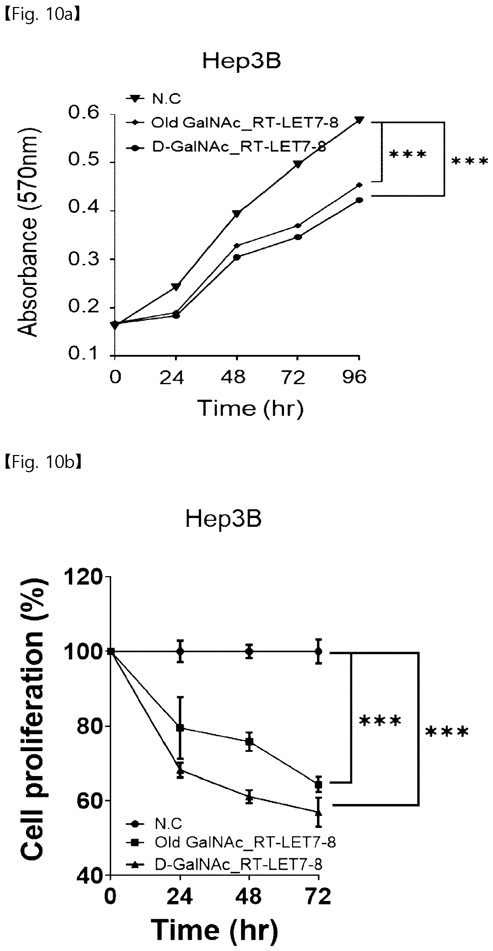

As a result of the MTT assay, it was found that D-GalNAc-RT-LET7-8 effectively inhibited tumorigenesis in liver cancer cells compared to Old-GalNAc-RT-LET7-8 (FIG. 10A). Subsequently, in the BrdU assay result, similarly to the MTT assay result, it was confirmed that the proliferation inhibition of Hep3B cells treated with D-GalNAc-RT-LET7-8 was significantly increased compared to Old-GalNAc-RT-LET7-8 (FIG. 10B).

### 6-3. Confirmation of regulation of macrophage phagocytosis by GalNAc-RT-LET7-8

To confirm changes in the expression of TSP1, a target protein of let-7i-5p, by lowering the expression of let-7i-5p, target microRNA of RT-LET7, Hep3B cells were treated with Old-GalNAc-RT-LET7-8 and D-GalNAc-RT-LET7-8, respectively, and then expression changes were confirmed through Western blot analysis.

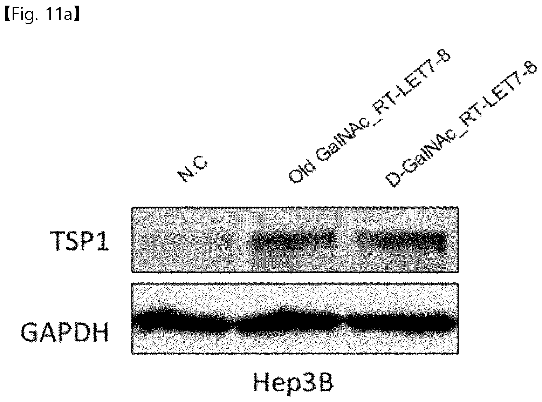

As a result, it was confirmed that in the case of D-GalNAc-RT-LET7-8, the expression of TSP1 was increased compared to Old-GalNAc-RT-LET7-8 (FIG. 11A).

Next, in order to confirm whether macrophages were able to phagocytose HCC, in vitro phagocytosis assay was performed using the method of Example 4.

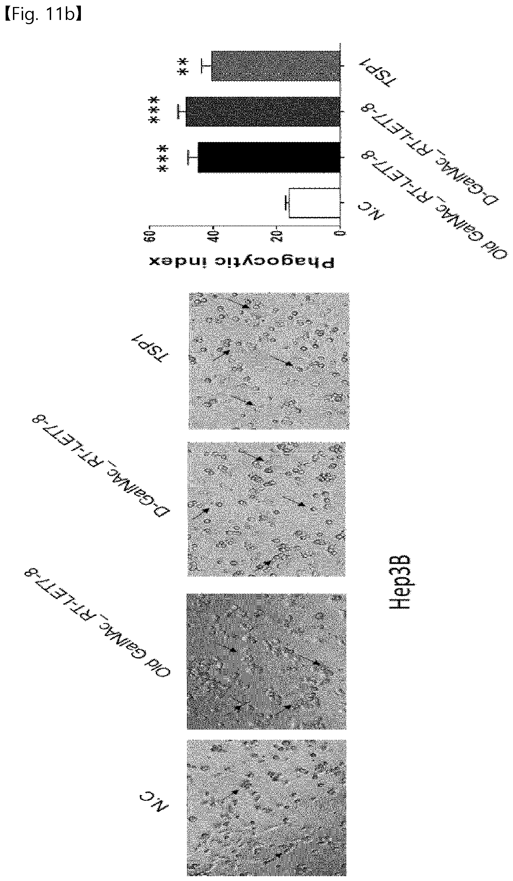

As a result, it was confirmed that in the case of D-GalNAc-RT-LET7-8, the phagocytosis activity of macrophages was further increased compared to Old-GalNAc-RT-LET7-8 (FIG. 11B).

Through these results, it was confirmed that when the modified RT-LET7-8 was loaded with the liver cell-targeted delivery system, the modified RT-LET7-8 increased delivery to the target organ, liver, thereby significantly improving the inhibition efficacy of Let-7i-5p expression in liver cells, the inhibition efficacy of tumorigenesis in liver cancer, and the activation efficacy of macrophage phagocytosis. In particular, it may be seen that in the liver cell-targeted delivery system, the modified RT-LET7-8 combined with D-GalNAc represented by Chemical Formula 2 significantly improves the above-mentioned prevention or treatment effect of liver cancer by RT-LET7-8.

## Claims

1. A nucleic acid molecule targeting Let-7i-5p, wherein the nucleic acid molecule is represented by a base sequence of SEQ ID NO: 1,
the nucleic acid molecule is a nucleic acid in which a nucleotide sequence is modified by 2'-O-methoxyethylation, and
a part or the entire backbone of the nucleic acid molecule is modified with phosphorothioate.

2. The nucleic acid molecule of claim 1, wherein among nucleotides in SEQ ID NO: 1, a backbone of first to fourth nucleotides from a 5' terminus is modified with phosphorothioate.

3. The nucleic acid molecule of claim 1, wherein among nucleotides in SEQ ID NO: 1, a backbone of first to fourth nucleotides from a 3' terminus is modified with phosphorothioate.

4. A nucleic acid molecule targeting Let-7i-5p, wherein the nucleic acid molecule is represented by a base sequence of SEQ ID NO: 1,
the nucleic acid molecule is a nucleic acid in which a nucleotide sequence is modified by 2'-O-methoxyethylation,
a part or the entire backbone of the nucleic acid molecule is modified with phosphorothioate, and
the nucleic acid molecule is combined with a liver cell-targeted moiety.

5. The nucleic acid molecule of claim 1, wherein the liver cell-targeted moiety is N-acetylgalactosamine (GalNAc) or galactosyl lipidoid nanoparticle (Gal-LNP).

6. The nucleic acid molecule of claim 5, wherein the N-acetylgalactosamine (GalNAc) is represented by Chemical Formula 1 or 2:

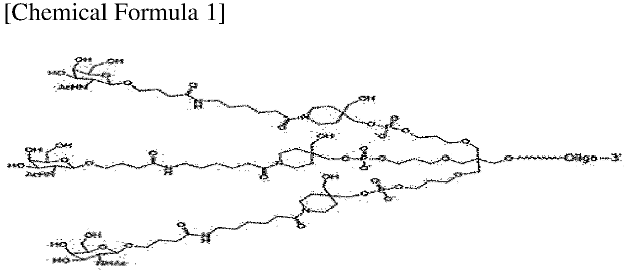

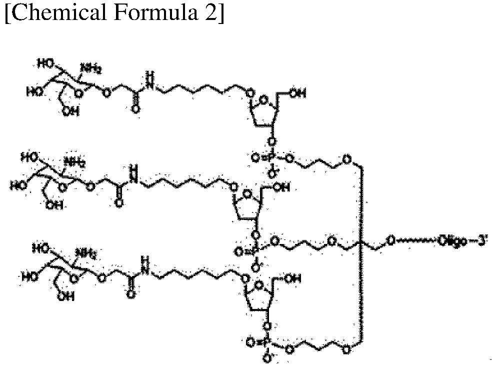

7. A pharmaceutical composition for preventing or treating liver cancer, the pharmaceutical composition comprising the nucleic acid molecule of any one of claims 1 to 6 as an active ingredient.

8. The pharmaceutical composition for preventing or treating liver cancer of claim 7, wherein the composition inhibits the expression of Let-7i-Sp.

9. The pharmaceutical composition for preventing or treating liver cancer of claim 7, wherein the composition increases the expression of thrombospondin-1 (TSP1).

10. The pharmaceutical composition for preventing or treating liver cancer of claim 7, wherein the composition increases phagocytosis activity of macrophages.

11. A checkpoint inhibitor for treating CD47-positive liver cancer, the checkpoint inhibitor comprising the nucleic acid molecule of any one of claims 1 to 6 as an active ingredient.

12. A method for treating liver cancer, the method comprising administering the pharmaceutical composition of claim 7 or the checkpoint inhibitor of claim 11 to a subject.
